# EUROPÄISCHE PATENTSCHRIFT

(11) **EP 0 617 928 B1**
(45) Veröffentlichungstag und Bekanntmachung des Hinweises auf die Patenterteilung: **16.07.1997**
(21) Anmeldenummer: 94104813.4
(22) Anmeldetag: 26.03.1994
(51) Int. Cl.: A61C 17/06

(54) **Absaugekanüle für zahnärztliche Zwecke**
Suction cannula for dental use
Canule d'aspiration pour l'usage dentaire

(30) Priorität: 30.03.1993 DE 4310225
(43) Veröffentlichungstag der Anmeldung: 05.10.1994
(73) Patentinhaber: Riewenherm, Ulrich Dr., D-33335 Gütersloh (DE)
(72) Erfinder: Riewenherm, Ulrich Dr., D-33335 Gütersloh (DE)
(74) Vertreter: Strauss, Hans-Jochen, Dipl.-Phys., Dr.

(56) Entgegenhaltungen:
- WO-A-92/12686
- CH-A- 101 276
- DE-A- 1 949 517
- DE-A- 2 709 116
- FR-A- 632 608
- FR-A- 2 560 038
- FR-A- 2 677 538
- US-A- 2 574 217

## Beschreibung

Die Erfindung betrifft eine Absaugekanüle für zahnärztliche Zwecke nach dem Oberbegriff des unabhängigen Anspruchs 1.

In der zahnärztlichen Praxis müssen Präparationen im Mundbereich durchgeführt werden, wobei mit hochtourigen Instrumenten gearbeitet wird. Die mit diesen Instrumenten bearbeiteten Zahnstellen bedürfen der Kühlung, wozu die Bohr- oder die Turbinenköpfe mit Wasserdüsen versehen sind, die auf das Behandlungsgebiet gerichtet sind. Für seine Arbeit benötigt der Zahnarzt Licht; um eine hinreichende Beleuchtung zu haben, wird das Lichtbündel der Behandlungsleuchte in die Mundhöhle gerichtet, wobei eine gute Öffnung des Mundes wesentlich ist, um die gewünschte Ausleuchtung zu erreichen. Um diese Ausleuchtung zu verbessern, bedient sich der Zahnarzt seines Mundspiegels, mit dem er auch den Fortgang der Arbeit überwachen kann. Mit diesem Mundspiegel kann der Zahnarzt dabei auch entweder die Zunge oder die Innenseite der Wange vor einer Verletzung durch das eingesetzte Arbeitsgerät schützen. Bei dieser Arbeit assistiert in aller Regel eine Helferin, die eine Absaugekanüle an das Behandlungsgebiet hält, um das versprühte Wasser abzusaugen. Mit dieser Absaugekanüle wird gleichzeitig die Innenseite der Wange oder die Zunge, die vom Mundspiegel des Zahnarztes nicht geschützt werden kann, dadurch geschützt, daß die Absaugekanüle gleichsam als Schutzschirm wirkend, den dem Behandlungsgebiet zugewandten Teil der Wange und den dem Behandlungsgebiet zugewandten Teil der Zunge zurückdrückt und so eine Gefährdung durch die Arbeitsgerätschaften des Zahnarztes ausschließt.

Nun steht nicht in allen Fällen eine assistierende Helferin zur Verfügung und der Zahnarzt muß ohne diese Assistenz auskommen; dabei treten zum einen Gefährdungen des Patienten auf, zum anderen ist der Zahnarzt gezwungen, die Arbeitsgerätschaften zu wechseln, wozu Handgriffe notwendig werden, die den Behandlungsablauf Stören.

Die DE-A-19 49 517 beschreibt eine Absaugesonde, wie im Oberbegriff des Anspruchs 1 dargestellt, mit einem in den Mund einführbaren, die Zahnleiste übergreifenden, in einem stumpfen Winkel an die Kanüle angefügten Ansatz mit Saugende, wobei der Ansatz einen ersten und einen zweiten Seitenflügel sowie ein diese Seitenflügel verbindendes Scheitelstück mit einer der Zahnleiste mit Zahn angepaßten Breite aufweist. Bei dieser Absaugesonde mündet das Saugende in einer Absaugeöffnung in dem ersten Seitenflügel, wobei Seitenflügel und Scheitelstück als schalenartig gewölbte "Wände" ausgebildet sind, und wobei die zweite, der der Absaugeöffnung zugeordnete Seitenwand gegenüberliegend angeordnete Seitenwand des "U" , gegenüber dieser ersten so in der Höhe versetzt ist, daß das freie Ende des zweiten Seitenflügels unterhalb des ersten Seitenflügels liegt. Diese Absaugesonde, die mit ihren schalenartig gewölbten lingualen und buccalen Seitenflügeln ein Zurückdrängen der Weichteile erlaubt, ist schwierig in der Handhabung und nur schwer herstellbar.

Hier setzt die Erfindung an, die die bekannten Absaugekanülen so weiterbilden soll, daß die vorgenannten Nachteile vermieden werden und der Zahnarzt auch ohne Assistenz einen Patienten ohne Gefährdung durch die Arbeitsgerätschaften behandeln kann und dabei von unnötigen Handgriffen zum Instrumentenwechsel freigehalten wird; darüber hinaus soll diese Weiterbildung einfach und sicher anwendbar und wirtschaftlich herstellbar sein.

Diese Aufgabe wird nun nach der Erfindung dadurch gelöst, daß der stumpfe Winkel "ε" des Ansatzes gegenüber dem dem Saugende der Absaugekanüle zwischen 80° und 135° liegt, und die im wesentlichen eben ausgebildeten Seitenflügel des Ansatzes in ihren Längserstreckungen einen Winkel "δ" von bis zu 30° derart bilden, daß das freie Ende der zweiten Seitenwand die Oberkante der ersten Seitenwand mindestens erreicht. Dabei sind deren Höhen derart, daß die Oberkanten beider Seitenflügel in Behandlungsposition mindestens an die Oberkanten der in der Zahnleiste befindlichen Zähne reichen. Diese Ausbildung schafft eine Absaugkanüle, die der Arbeitsweise des Zahnarztes angepaßt ist, die er einfach so halten kann, daß die Weichteile in der Umgebung des Behandlungsgebietes, die Wange einerseits und die Zunge andererseits, besonders geschützt sind; dabei kann er mit der anderen Hand das Mundstück halten und zur Präparation so führen, das er seine Arbeit ungestört ausführen kann. Dabei ist diese Ausführungform wegen der im wesentlichen eben ausgeführten Wänden auch einfach und wirtschaftlich herstellbar.

In einer vorteilhaften Ausführungsform ist der Ansatz einstückig an die vorzugsweise als Kunststoff-Spritzgußteil ausgebildete Kanüle angeformt; alternativ ist der Ansatz als Kunststoff-Spritzgußteil so ausgebildet, daß er an die Absaugeöffnung der Kanüle ansetzbar, wozu ein das Saugende der Kanüle formschlüssig aufnehmender Aufnahme-Stutzen an dem Ansatz vorgesehen oder das Saugende der Absaugekanüle so ausgebildet ist, daß es formschlüssig in eine korrespondierende Ausnehmung in der ersten Seitenwand des Ansatzes einsetzbar ist. Rastmittel, etwa Rastsegmente oder Rastnasen im Zusammenwirken mit ansprechenden Rastnuten oder Rastausnehmungen sichern dabei den an die Kanüle angesetzten Ansatz. Während die erste der beiden Ausführungsformen als "Einmal-Kanüle" anzusprechen ist, ist bei der zweiten Ausführungsform der Ansatz austauschbar, so daß hier die üblichen Edelstahlkanülen einsetzbar sind, an die die Zahnleiste mit dem/den Zahn/Zähnen übergreifenden "U" angesetzt und von Patient zu Patient gewechselt werden können.

Für die Ausbildung als Kunststoff-Teil wird ein hygienisch unbedenklicher und sterilisierbarer Kunststoff vorgesehen, der - wenn spritzgießfähig - auch die Herstellung von Kanüle mit Ansatz oder des an die Kanüle ansetzbaren Ansatzes im Spritzgußverfahren erlaubt. Diese so ausgebildete Absaugekanüle kann der Zahnarzt in die Mundhöhle einführen, wobei der Ansatz infolge des stumpfen Winkels beidseits des zu behandelnden Zahnes liegt und das Scheitelstück die Zahnleiste übergreift. Damit wird das Behandlungsgebiet abgegrenzt. Bei der Form des Ansatzes liegen die beiden Seitenwände im wesentlichen parallel zur Zahnleiste, wobei ein Arbeiten in allen vier Quadranten der Kiefer ermöglicht wird. Die beiden Seitenwände schützen dabei die dem Behandlungsgebiet benachbarten Weichteile vor einer Gefährdung durch die Behandlungswerkzeuge; dabei bleibt das für den Patienten angenehme Zungenspiel erhalten, so daß Schluckbewegungen möglich sind. Sowohl die jeweiligen, der Okklusion abgewandten Kanten der Seitenwände als auch die der Zahnleiste zugewandte Kante des Scheitelstückes können einzeln oder gemeinsam gegen den Kiefern- bzw. Gaumenboden abgestützt werden; damit wird auch dem Zahnarzt die Möglichkeit gegeben, einen Abstützpunkt für die das Behandlungswerkzeug führende Hand zu finden, was die Genauigkeit der Führung des Behandlungswerkzeuges gegenüber einer Freihand-Führung erheblich verbessert. Zum anderen ist durch die Abgrenzung des Behandlungsgebietes die Luftströmung im Behandlungsgebiet deutlich erhöht, was die Effizienz der Absaugung erhöht. Dabei ist die Absaugeöffnung in der der Kanüle zugeordneten Seitenwand so angeordnet, daß ihre Mitte etwa in Äquatorhöhe des zu behandelnden Zahnes liegt. Dabei versteht es sich von selbst, daß für unterschiedliche Kiefergrößen, bei Bedarf auch für die einzelnen Quadranten oder aber für Rechts- oder Linkshändigkeit des behandelnden Zahnarztes bei Erhalt der U-förmigen Grundform unterschiedliche Ausformungen der Absaugekanüle möglich sind.

In einer bevorzugten Ausführungsform weist der Ansatz zumindest im Bereich des Scheitelstückes eine zum Innenraum des offenen "U" des Ansatzes gerichtete Spiegelfläche auf. Mittels dieser Spiegelfläche auf dem Scheitelstück sowie ggf. auf einer oder beiden Seitenflächen wird zum einen Licht "gefangen" und auf das Behandlungsgebiet zurückgeworfen, zum anderen wird dem Zahnarzt die gewohnte Beobachtungsmöglichkeit gegeben, ohne daß es eines zusätzlichen Mundspiegels bedarf, da die so ausgebildete Absaugekanüle wie ein Mundspiegel geführt werden kann. In einer vorteilhaften Ausführungsform ist die Spiegelfläche gebildet durch auf den Innenseiten zumindest einer der Seitenwände und/oder des Scheitelstückes vorgesehenen verspiegelten Flächen, die aufgedampft oder die galvanisch aufgebracht sein können. Vorteilhaft ist es, wenn diese Spiegelflächen durch Plasma-Ionen-Auftragsverfahren aufgebracht werden. Dazu werden mit Mitteln der Gasentladung Ionen erzeugt, die auf die zu metallisierenden Flächen aufprallen und sich dort niederschlagen. Vorteilhaft wird dabei zunächst eine Unterschicht etwa aus Kupfer aufgetragen, auf der dann die spiegelnde Schicht aufgebracht wird, etwa als Titan- oder Chromschicht. Dabei versteht es sich von selbst, daß die verspiegelten Innenflächen hinreichend plan ausgebildet sind, was durch eine dieser Bedingung angepaßten Herstellung erreicht werden kann.

Durch diese Anordnung des Spiegels bzw. der Spiegel wird zum einen das von der Behandlungsleuchte einfallende Licht auf die zu behandelnde Stelle gelenkt, so daß die gewünschte Ausleuchtung sicher gestellt ist, wobei es sich von selbst versteht, daß die Ausleuchtung von dem Ort der Präparation am Zahn abhängig ist, zum andern wird dem Zahnarzt die Möglichkeit gegeben, das Behandlungsgebiet zu inspizieren, so daß er Restdefekte, insbesondere Rest-Karies zu erkennen in der Lage ist. Dabei kann der Zahnarzt, der die mit dem Ansatz versehene Absaugekanüle frei hält, den Spiegel so einrichten, daß die Ausleuchtung des Behandlungsgebietes optimal für ihn ist. Es versteht sich dabei von selbst, daß diese Ausleuchtung und die Beobachtungsmöglichkeit auch dadurch erzielt werden kann, daß ein üblicher Spiegel im Bereich des Scheitelstückes und/oder der Seitenwände vorgesehen wird.

Vorteilhaft ist dabei die Ausbildung des Ansatzes als sich öffnendes "U", wobei der Winkel α der beiden Seitenflächen des "U" gegeneinander im Bereich bis zu 40° liegt. Durch diese Öffnung erhält der Zahnarzt genügend Raum zum Präparieren auch an seitlichen Zahnflächen.

Um einen verbesserten Zugang zum Behandlungsgebiet zu erreichen, ist es vorteilhaft, wenn zumindest die der mit der Absaugeöffnung versehenen ersten Seitenwand gegenüberliegend angeordnete zweite Seitenwand um einen Winkel β von bis zu etwa 20° nach außen geneigt ist.

Um die unterschiedlichen Formen der Unterkiefer-Wangentasche und des Zungenboden oder der Oberkiefer-Wangentasche und des Gaumens zu berücksichtigen, ist es weiter vorteilhaft, wenn die äußere, der mit der Absaugeöffnung versehenen ersten Seitenwand gegenüberliegend angeordnete zweite Seitenwand mit der Längserstreckung der ersten einen Winkel δ von höchstens 30° derart bildet, daß das freie Ende der zweiten Seitenwand die Oberkante der ersten Seitenwand mindestens erreicht, wenn nicht sogar überragt.

Vorteilhaft ist dabei, wenn das Scheitelstück und/oder die zweite Seitenwand des Ansatzes eine gegenüber der der Absaugeöffnung zugeordneten, ersten Seitenwand verringerte Breite aufweist. Diese Ausführungsform berücksichtigt, daß die der Wange zugewandte Seitenfläche des Ansatzes der Absaugekanüle tiefer in die Wangentasche eingreifen kann, als die der Zunge zugewandte Seitenfläche. Bei der Behandlung im Gebiet der Prämolaren kann das Scheitelstück ohne weiteres über einen hinter dem zu behandelnden Zahn liegenden Zahn geführt werden, wobei das Scheitelstück sogar auf diesem Zahn abgestützt werden kann; bei Behandlungen im Bereich des hintersten Molars übergreift das Scheitelstück die Zahnleiste.

Vorteilhaft ist weiter der untere Rand zumindest der der Absaugeöffnung zugeordneten Seitenwand wulstartig ausgebildet und vorzugsweise zu der freien Seitenwand hin umgebogen. Diese Wulst legt sich in der Tiefe der Wangentasche gegen ihren Grund oder gegen die Zahnleiste, so daß hier ein Abschluß gegeben ist, der das Behandlungsgebiet eingrenzt. Um dem Patienten eine Behandlung erträglicher zu gestalten, ist es weiter vorteilhaft, wenn der obere Rand des Scheitelstückes des Ansatzes eine Beißwulst aufweist. Auf diese Beißwulst kann der Patient "aufbeißen" und so die Muskulatur entlasten. Weiter weist in einer vorteilhaften Weiterbildung der obere Rand des Scheitelstückes des Schutzansatzes eine Beißwulst auf.

Mit der so geschaffenen Absaugekanüle wird dem Zahnarzt ein Instrument an die Hand gegeben, daß ihm die Möglichkeit eröffnet, ohne Helferin auch schwierige Präparationen vornehmen zu können, wobei der Ansatz gegen den Zungenboden bzw. gegen den Gaumen abgestützt werden kann, und so ein Fixpunkt geschaffen wird, der dem Zahnarzt zum Abstützen der das Handstück führenden Hand dienen kann. Der Patient empfindet dabei ebenfalls Erleichterung, weil eine immer in seinem Gesichtsfeld bewegte Hand der Helferin entfällt, weil der Weichteile-Schutz ohne starke Einschränkung der Bewegbarkeit der Zunge verbessert ist, und weil die sehr effektive Absaugung einen größeren Anteil des zur Kühlung eingesetzten Wassers erfaßt, so daß sich weniger Wasser in tiefer gelegenen Partien der Mundhöhle sammeln kann, die einmal weniger zum Schlucken Anlaß geben und die zum anderen wegen der erhaltenen Bewegbarkeit der Zunge ohne weiteres geschluckt werden können. Schließlich kann der Ansatz auch so ausgebildet werden, daß ein Kofferdamm-Gummi angesetzt werden kann, das sich dann ohne weiteres schützend um das Behandlungsgebiet legt, und der allein durch den auf die Absaugekanüle ausgeübten Druck gespannt gehalten wird. Dazu werden die Seitenwände des Ansatzes in einfacher Weise mit Nocken versehen, in die das Kofferdamm-Gummi eingehängt und gespannt gehalten werden kann.

Das Wesen der Erfindung wird durch die in den Figuren 1 bis 5 dargestellten Ausführungsformen beispielhaft näher beschrieben; dabei zeigen
- Fig. 01:: Eine perspektivische Ansicht des Saugendes einer Absaugekanüle mit Ansatz (Behandlungsgebiet angedeutet);
- Fig. 02:: Eine Aufsicht der Absaugekanüle nach Fig. 1;
- FIG. 03:: Eine Seitansicht der Absaugekanüle nach Fig. 1;
- Fig. 04:: Eine Frontansicht der Absaugekanüle nach Fig. 1;
- Fig. 05:: Ansatz mit seitlichen Zusatzspiegeln und Rastnasen für Kofferdamm-Gummi;

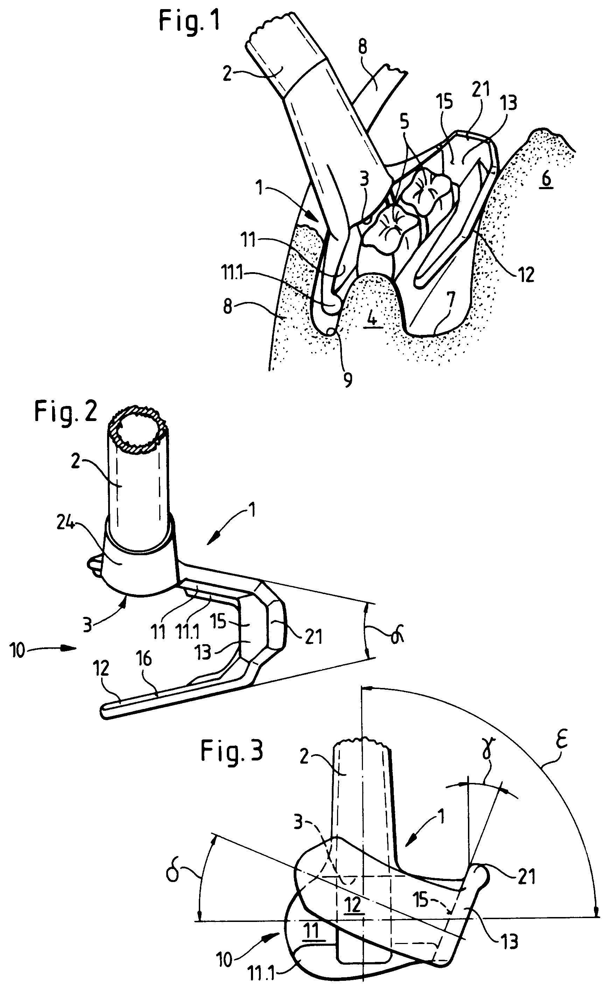

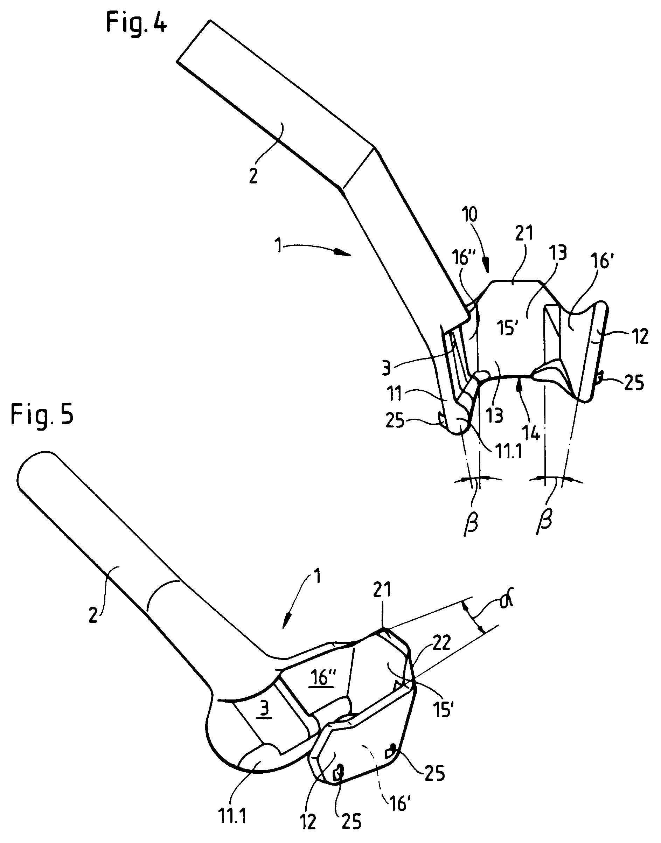

Die Figur 1 zeigt eine perspektivische Darstellung einer Ausführungsform der Absaugekanüle 1 für zahnärztliche Zwecke, bei der das Saugende 2 mit dem Ansatz 10 in den Quadranten IV des Unterkiefers eingesetzt ist und dort die Zahnleiste 4 mit den Zähnen 5 übergreift, wobei das der Seitenwand 11 des Ansatzes zugeordnete Saugende 2 der Kanüle 1 diese durchdringt und in einer Saugöffnung 3 mündet. Der Ansatz 10 legt sich mit seinen beiden Seitenwänden 11 und 12 beidseits neben die Zahnleiste 4, so daß die Zähne 5 zwischen den beiden Seitenwänden 11 und 12 des Ansatzes 10 liegen, wobei dieser gegen den Zungenboden 7 und/oder gegen den Grund 9 der Wangentasche 8 abgestützt ist (wobei es sich von selbst versteht, daß die analogen Situationen in den anderen Quadranten entsprechend möglich sind). Die gleiche Absaugekanüle 1, allerdings ohne eingezeichnete Zahnleiste mit Zähnen und ohne die umgebenden Weichteile, zeigen Fig. 2 in Seitansicht, Fig. 3 in Aufsicht und Fig. 4 in Frontansicht.

Die Absaugekanüle 1 ist dabei in der üblichen Weise ausgebildet, sie geht mit einer Krümmung in den (nicht näher dargestellten) Schlauchanschluß über, und das andere Ende der Absaugekanüle 1 in das Saugende 2 mit der Absaugeöffnung 3. Der an die Absaugekanüle 1 mit der Absaugeöffnung 3 angeformte (oder in einer Weiterbildung nach Fig. 2 angesteckte) Ansatz 10 ist im wesentlichen "U"-förmig ausgebildet mit einer die Absaugeöffnung 3 enthaltenden ersten Seitenwand 11 und einer gegenüberliegenden zweiten Seitenwand 12 versehen, die durch ein Scheitelstück 13 des Ansatzes 10 miteinander verbunden sind, und deren freies Ende 12.1 die Öffnung des "U" begrenzt. Während in der Ausführungsform nach Figur 1 (sowie auch Fig. 3 und 4) der Ansatz 10 an die Absaugekanüle 1 angeformt ist, wobei diese Teile vorteilhaft als Kunststoff-Spritzgußteile ausgebildet sind, ist in der in Figur 2 dargestellten Ausführungsform der Ansatz 10 als getrenntes Teil hergestellt und mit einer Steckhülse 24 versehen, in das das Saugende 2 der Absaugekanüle 1 formschlüssig einführbar ist. Dabei können auch kraft- oder formschlüssige Sicherungen vorgesehen sein, die die Verbindung Absaugekanüle 1 und Ansatz 10 fixieren.

Die Breite der beiden Seitenwände 11 und 12 ist dabei zumindest so groß, wie die übliche Höhe der Zahnleisten 4 der Kiefer mit den darin befindlichen Zähnen 5, so daß beide Seitenwände 11 und 12 in Behandlungsposition mindestens bis an die Oberkanten der in der Zahnleiste 4 befindlichen Zähne 6 reicht und die beiden Seitenwände 11 und 12 auf den beiden Seiten der Zahnleiste 4 zu liegen kommen. Um diese Position einhalten zu können, ist das Scheitelstück 13 des Ansatzes 10 mit einer Einnehmung 14 versehen, so daß dieser Scheitel ohne Schwierigkeiten über die Zahnleiste 4 ggf. auch über einen darin befindlichen Zahn 5 geführt werden kann. Die Tiefe der Einnehmung 14 ist dabei üblichen Zahnhöhen angepaßt. Um einen übermäßigen Druck im Bereich der Auflagestellen (besonders bei metallischen Ansätzen 10 zu vermeiden, werden die unteren Randbereiche vorteilhaft mit einer Wulst 11.1 bzw. 12.1 versehen, die durch Anformen oder bei metallischen Ansätzen 10 auch durch Umbördeln erhalten werden. Der Ansatz 10 weist dabei gegenüber der Absaugekanüle eine Winkelstellung entsprechend dem Winkel ε (Fig. 3) von etwa 80° bis 135° auf. Die Absaugekanüle kann dabei so in den Mund des Patienten eingeführt werden, daß zur Präparation eines Zahnes beide Seitenwände 11 und 12 des Ansatzes 10 neben der Zahnleiste 4 des Kiefern-Quadranten, in dem sich der zu präparierende Zahn 5 befindet, liegen, wobei jeder Kiefer-Quadrant erreicht werden kann. Durch diese Ausbildung wird weiter das Präparationsgebiet eingegrenzt; trotzdem kann der Zahnarzt dabei die Präparationsstelle frei erreichen, wobei mit dem Handstück-Kopf eine weitere Abgrenzung des Präparationsgebietes erreicht wird. Dabei hält der Zahnarzt die Absaugekanüle 1 mit der einen und das Handstück mit dem Behandlungswerkzeug mit der anderen Hand und kann so sowohl dieses Handstück als auch die Absaugekanüle so einrichten, wie es die örtlichen Gegebenheiten des jeweiligen Behandlungsgebietes in einem der vier Quadranten erfordern.

Zur Ausleuchtung des Behandlungsgebietes ist die Innenfläche des Scheitelstückes 13 mit der Spiegelfläche 15 versehen, die als metallisierte Fläche ausgebildet ist. Gleiches gilt auch für die Seitenwand 12, die der mit der Saugöffnung 3 versehenen Seitenwand 11 gegenüberliegend angeordnet ist, wobei hier der Spiegel 16 etwa rechtwinklig zum Spiegel 15 des Scheitelstückes 13 liegt. Der Spiegel 15 des Scheitelstückes 13 hat dabei gegenüber dem Ansatz 10 eine nach außen gerichtete Neigung (Winkel γ, Fig. 3) bis etwa 20°, so daß einfallendes Licht gut in das Behandlungsgebiet gespiegelt wird. Entsprechend ist die Neigung des in der Seitenwand 12 vorgesehenen Spiegels 16 so, daß eine auswärts gerichtete Neigung bis etwa 20° vorgesehen ist (Winkel β Fig. 4). Die Figur 4 läßt (durch den angedeuteten Übergangsbereich zwischen der Innenseite des Scheitelstückes 13 und der beiden Seitenwänden 11 und 12 die Öffnung des "U" erkennen, dessen Winkel α (Fig. 2) in der Praxis im Bereich von bis zu 40° liegt. In der Ausführungsform nach Figur 4 sind weiter die Innenflächen der Seitenwände verspiegelt; dadurch entstehen die Spiegel 15' sowie 16' und 16'' (hier perspektivisch stark verkürzt), wobei es sich von selbst versteht, daß diese Flächen von der Herstellung her die notwendige Planheit aufweisen. Zur Vermeidung von optischen Verzerrungen sind die winkligen oder abgerundeten Übergänge von der Verspiegelung ausgenommen. Wird dabei die Innenseite des Scheitelstückes 13 "geknickt" ausgebildet, entsteht ein weiterer Spiegel 15'', so daß sich insgesamt 4 Spiegel ergeben, die zum einen das Licht in das Behandlungsgebiet lenken, die aber auch zum anderen dem Zahnarzt die Übersicht über das Behandlungsgebiet mit seiner Umgebung gibt, die Restdefekte, etwa Rest-Karies zu erkennen erlauben. Vorteilhaft ist eine Gesamt-Metallisierung, wobei die Bereiche der Innen- und der Außenflächen, die nicht spiegeln sollen, mattiert ausgeführt sind. Dieser metallische Gesamt-Überzug ist im Hinblick auf Hygienefragen vorteilhaft, er überzieht den Ansatz lückenlos und erlaubt so eine einwandfreie Sterilisierung.

Die Figur 5 zeigt nochmals ein perspektivisches Schema eines Ansatzes 10, dessen Seitenwände 11 und 12 mit dem Scheitelstück 13 ein offenes "U" bilden, wobei die Seitenflächen das "U" divergieren, so daß sich seine Öffnung erweitert. Das Scheitelstück 13 ist mit einer hinteren Auslegung 22 so ausgeformt, daß die Bißwulst 21 hinreichend Platz findet. Die Innenseiten der Seitenwände und des Scheitelstükkes 13 -hier im Gegensatz zu Figur 4 durchgehend plan dargestelltensind verspiegelt und mit den Spiegels 15', 16' und 16'' versehen, die alle drei zum Innenraum des Ansatzes gerichtet sind. Auf der Außenseite der Seitenwände (hier ist nur die zweite Seitenwand 12 sichtbar) sind die Einhängenasen 25 für das Kofferdamm-Gummi vorgesehen. Die Lage dieser Einhängenasen ist derart, daß bei in den Mund eingesetztem Ansatz die Zahnleiste mit den Zähnen das Kofferdamm-Gummi spannen und so eine vorteilhafte Halterung dafür darstellen.

## Patentansprüche

1. Absaugekanüle (1) für zahnärztliche Zwecke mit einem Absaugerohr (2) mit Saugende, an das ein in den Mund einführbarer, die Zahnleiste übergreifender Ansatz (10) mit Saugende (2) in einem Winkel "ε" angefügt ist, wobei der Ansatz etwa U-förmig ausgebildet einen ersten und einen zweiten Seitenflügel sowie ein diese Seitenflügel verbindendes Scheitelstück mit einer der Zahnleiste (4) mit Zahn (5) angepaßten Breite aufweist, wobei das Saugende in einer Absaugeöffnung in dem ersten Seitenflügel mündet, wobei Seitenflügel (11, 12) und Scheitelstück (13) als Wände ausgebildet sind, und wobei die zweite, der der Absaugeöffnung (3) zugeordneten Seitenwand (12; 11) gegenüberliegend angeordnete Seitenwand (12; 11) des "U" (10), gegenüber dieser ersten in der Höhe versetzt ist, **dadurch gekennzeichnet**, daß der Winkel "ε" des Ansatzes (10) gegenüber dem Saugende (2) der Absaugkanüle (1) zwischen 80° und 135° liegt, und die im wesentlichen eben ausgebildeten Seitenflügel (11, 12) des Ansatzes (10) in ihren Längserstreckungen einen Winkel "δ" von bis zu 30° derart bilden, daß das freie Ende der zweiten Seitenwand (12) die Oberkante der ersten Seitenwand (11) mindestens erreicht, wobei deren Höhen derart sind, daß die Oberkanten beider Seitenflügel in Behandlungsposition mindestens an die Oberkanten der in der Zahnleiste (4) befindlichen Zähne (5) reichen.

2. Absaugekanüle nach Anspruch 1, **dadurch gekennzeichnet**, daß der Ansatz (10) einstückig an die vorzugsweise als Kunststoff-Spritzgußteil ausgebildete Kanüle (1) angeformt ist.

3. Absaugekanüle nach Anspruch 1, **dadurch gekennzeichnet**, daß der vorzugsweise als Kunststoff-Spritzgußteil ausgebildete Ansatz (10) an die Absaugeöffnung (3) der Kanüle (1) ansetzbar ist.

4. Absaugekanüle nach Anspruch 1 bis 3, **dadurch gekennzeichnet**, daß der Ansatz (10) zumindest im Bereich des Scheitelstückes (13) eine zum Innenraum des offenen "U" des Ansatzes (10) gerichtete, vorzugsweise um einen Winkel "γ" bis zu etwa 20° nach außen geneigte Spiegelflächen (15; 16) aufweist.

5. Absaugekanüle nach Anspruch 4, **dadurch gekennzeichnet**, daß die Spiegelfläche/-n (15, 16) gebildet ist/sind durch auf den Innenseiten zumindest einer der Seitenwände (11; 12) und/oder des Scheitelstückes (13) vorgesehenen verspiegelten Flächen (15', 16', 16''), wobei die verspiegelten Flächen vorzugsweise von einem Metallauftrag wie Titan- oder Chromauftrag gebildet sind.

6. Absaugekanüle nach einem der Ansprüche 1 bis 5, **dadurch gekennzeichnet**, daß das von der ersten Seitenwand (11), der zweiten Seitenwand (12) und dem Scheitelstück (13) gebildete "U" eine sich öffnende Form aufweist, mit einem Öffnungswinkel "α" bis zu etwa 40°.

7. Absaugekanüle nach einem der Ansprüche 1 bis 6, **dadurch gekennzeichnet**, daß zumindest die der mit der Absaugeöffnung (3) versehenen ersten Seitenwand (11) gegenüberliegend angeordnete zweite Seitenwand (12) um einen Winkel "β" von bis zu etwa 20° nach außen geneigt ist.

8. Absaugekanüle nach einem der Ansprüche 1 bis 7, **dadurch gekennzeichnet**, daß das Scheitelstück (13) des "U" (10) und/ oder die der ersten Seitenwand (11) mit Absaugeöffnung (3) gegenüberliegend angeordnete zweite Seitenwand (12) eine gegenüber der ersten Seitenwand (11) verringerte Breite aufweist.

9. Absaugekanüle nach einem der Ansprüche 1 bis 9, **dadurch gekennzeichnet**, daß der untere Rand zumindest der der Absaugeöffnung (3) zugeordneten ersten Seitenwand (11) als Auflagewulst (11.1) ausgebildet und vorzugsweise zu der zweiten Seitenwand (12) hin umgebogen ist, und daß vorzugsweise der obere Rand des Scheitelstückes (13) des Ansatzes (10) eine Beißwulst (21) aufweist.

## Claims

1. A suction tube system (1) for dental purposes having a suction tube (2) with a suction end, attached to which is an extension piece (10) with a suction end (2) arranged at an angle "ε" which can be inserted in the mouth to bridge the row of teeth, whereby the extension-piece is approximately of a U-shaped design and has a first and a second side-piece as well as a top-piece linking these side-pieces and being of a width which matches the row of teeth (4) and tooth (5), wherein the suction end opens at a suction orifice in the first side-piece, wherein the side-pieces (11, 12) and top-piece (13) are walls and wherein the second side wall (12; 11) arranged opposite the side wall (12; 11) of the "U" (10) which has the suction orifice (3) is arranged at a height which is offset relative to this first oppositely arranged side wall (12; 11) of the "U" (10), **characterised in that** the extension-piece (10) and the suction end (2) of the suction-tube system (1) subtend an angle "ε" of between 80° and 135° and the essentially flat side-pieces (11, 12) of the extension-piece (10) subtend an angle "δ" of up to 30° in their longitudinal extensions such that the free end of the second side wall (12) reaches at least the top edge of the first side wall (11), the heights thereof being such that the upper edges of both side walls reach at least the upper edges of the teeth (5) in the row of teeth (4) when in the operating position.

2. A suction-tube system as claimed in claim 1, **characterised in that** by preference, the extension-piece (10) is integrally moulded onto the suction-tube system (1) which is a plastics injection-moulded member.

3. A suction-tube system as claimed in claim 1, **characterised in that** the extension-piece (10), which is preferably a plastics injection-moulded member, can be attached to the suction orifice (3) of the suction-tube system (1).

4. A suction-tube system as claimed in claims 1 to 3, **characterised in that** the extension-piece (10) has a mirrored surface (15; 16), at least in the region of the top-piece, (13) outwardly inclined at an angle "α" of up to 20° directed towards the interior space of the open "U" of the extension-piece (10).

5. A suction-tube system as claimed in claim 4, **characterised in that** the mirrored surfaces(s) (15, 16) is/are formed by reflective surfaces (15', 16', 16'') provided on the internal faces of at least one of the side walls (11; 12) and/or the top-piece (13), the mirrored surfaces being made by preference of a layer of metal such as titanium or chromium.

6. A suction-tube system as claimed in one of claims 1 to 5, **characterised in that** the "U" formed by the first side wall (11), the second side wall (12) and the top-piece (13) is of a tapered shape opening at an angle "α" up to about 40°.

7. A suction-tube system as claimed in one of claims 1 to 6, characterised in that at least the second side wall (12) arranged opposite the first side wall (11) which has the suction orifice (3) is outwardly inclined at an angle "β" of up to about 20°.

8. A suction-tube system as claimed in one of claims 1 to 7, **characterised in that** the top-piece (13) of the "U" (10) and/or the second side wall (12) arranged opposite the first side wall (11) which has the suction orifice (3) is of a smaller width than the first side wall (11).

9. A suction-tube system as claimed in one of claims 1 to 9, **characterised in that** at least the lower edge of the first side wall (11) with the suction orifice (3) is a bulbous support surface (11.1) preferably bending round towards the second side wall (12) and the upper edge of the top-piece (13) of the extension-piece (10) preferably has a gripping bead (21).

## Revendications

1. Canule d'aspiration (1) à usage dentaire, comprenant un tube d'aspiration (2) à extrémité d'aspiration, auquel se raccorde, suivant un angle 〈〈 ε 〉〉, un embout (10) à extrémité d'aspiration (2) qui peut être introduit dans la bouche et chevauche le maxillaire, l'embout, réalisé approximativement en forme de U, comportant une première aile latérale et une seconde aile latérale, ainsi qu'une partie formant sommet qui relie ces ailes latérales et a une largeur adaptée au maxillaire (4) à dent (5), tandis que l'extrémité d'aspiration débouche par un orifice d'aspiration dans la première aile latérale, que les ailes latérales (11, 12) et la partie formant sommet (13) sont réalisées sous forme de parois et que la seconde paroi latérale (12 ; 11) du "U" (10) qui est disposée de façon à faire face à la paroi latérale (12 ; 11) associée à l'orifice d'aspiration (3) est décalée en hauteur vis-à-vis de cette première paroi latérale, caractérisée en ce que l'angle "ε" de l'embout (10) vis-à-vis de l'extrémité d'aspiration (2) de la canule d'aspiration (1) est compris entre 80° et 135° et en ce que les ailes latérales (11, 12) de l'embout (10), réalisées avec une forme qui, pour l'essentiel, est plane, forment un angle "δ" pouvant atteindre 30° entre les directions longitudinales suivant lesquelles elles s'étendent, d'une façon telle que l'extrémité libre de la seconde paroi latérale (12) atteint au moins l'arête supérieure de la première paroi latérale (11), tandis que leurs hauteurs sont telles que, dans une position de traitement, les arêtes supérieures des deux ailes latérales atteignent au moins les arêtes supérieures des dents (5) se trouvant dans le maxillaire (4).

2. Canule d'aspiration selon la revendication 1, caractérisée en ce que l'embout (10) est formé d'une seule pièce sur la canule (1) réalisée de préférence sous forme d'une pièce moulée par injection en matière plastique.

3. Canule d'aspiration selon la revendication 1, caractérisée en ce que l'embout (10), réalisé de préférence sous forme d'une pièce moulée par injection en matière plastique, peut être monté sur l'orifice d'aspiration (3) de la canule (1).

4. Canule d'aspiration selon l'une des revendications 1 à 3, caractérisée en ce qu'au moins dans la zone de la partie formant sommet (13), l'embout (10) comporte une ou des surfaces réfléchissantes (15 ; 16) faisant face à la cavité du "U" ouvert de l'embout (10) et inclinées de préférences d'un angle "γ" pouvant atteindre 20° environ vers l'extérieur.

5. Canule d'aspiration selon la revendication 4, caractérisée à ce que la ou les surfaces réfléchissantes (15, 16) sont formées de surfaces à dépôt réfléchissant (15', 16', 16'') prévues sur les faces intérieures d'au moins l'une des parois latérales (11 ; 12) et/ou de la partie formant sommet (13), les surfaces à dépôt réfléchissant étant de préférence formées d'un dépôt métallique tel qu'un dépôt de titane ou de chrome.

6. Canule d'aspiration selon l'une des revendications 1 à 5, caractérisée en ce que le "U" formé de la première paroi latérale (11), la seconde paroi latérale (12) et la partie formant sommet (13) présente une forme qui va en s'ouvrant, avec un angle d'ouverture "α" pouvant atteindre 40° environ.

7. Canule d'aspiration selon l'une des revendications 1 à 6, caractérisée en ce qu'au moins la seconde paroi latérale (12) qui est disposée face à la première paroi latérale (11) pourvue de l'orifice d'aspiration (3) est inclinée d'un angle "β" pouvant atteindre 20° environ vers l'extérieur.

8. Canule d'aspiration selon l'une des revendications 1 à 7, caractérisée en ce que la partie formant sommet (13) du "U" (10) et/ou la seconde paroi latérale (12) disposée de façon à faire face à la première paroi latérale (11) à orifice d'aspiration (3) présentent une largeur plus étroite que la première paroi latérale (11).

9. Canule d'aspiration selon l'une des revendications 1 à 9, caractérisée en ce que le bord inférieur d'au moins la première paroi latérale (11) associée à l'orifice d'aspiration (3) est réalisé sous forme d'un bourrelet d'appui (11.1) et présente de préférence une forme bombée en direction de la seconde paroi latérale (12) et en ce que le bord supérieur de la partie formant sommet (13) de l'embout (10) comporte de préférence un bourrelet à mordre (21).
